# EUROPÄISCHE PATENTSCHRIFT

(11) **EP 1 974 712 B1**
(45) Veröffentlichungstag und Bekanntmachung des Hinweises auf die Patenterteilung: **27.10.2010**
(21) Anmeldenummer: 07005396.2
(22) Anmeldetag: 15.03.2007
(51) Int. Cl.: A61K 6/00, A61K 6/083

(54) **Dentalmaterialien auf der Basis von radikalisch polymerisationsfähigen, N,O-funktionalisierten Acrylsäure-hydroxamiden**
Dental material based on radically polymerisable N,O-functionalised acrylic acid hydroxamides
Matériau dentaire a base des hydroxyamides d'acide acrylique pouvant être polymérisés de manière radicale et fonctionnalisés au N,O

(43) Veröffentlichungstag der Anmeldung: 01.10.2008
(73) Patentinhaber: Ivoclar Vivadent AG, 9494 Schaan (LI)
(72) Erfinder: Moszner, Norbert, Prof. Dr., 9495 Triesen (LI); Lamparth, Iris, Dr., 9472 Grabs (CH); Fischer, Urs Karl, 9320 Arbon (CH); Zeuner, Frank Dr., 9488 Schellenberg (LI); de Meijere, Armin, Prof. Dr., 37077 Göttingen (DE); Rheinberger, Volker M., Dr., 9490 Vaduz (LI)
(74) Vertreter: UEXKÜLL & STOLBERG

(56) Entgegenhaltungen:
- WO-A-03/035013

## Beschreibung

Die vorliegende Erfindung betrifft die Verwendung von radikalisch polymerisationsfähigen, N,O-funktionalisierten Acrylsäure-hydroxamiden als Vernetzer und/oder Haftmonomere für Dentalmaterialien zur Herstellung von Adhäsiven, Beschichtungen oder Kompositen.

Amide der Acryl- bzw. Methacrylsäure sind als Comonomere für die Herstellung von Polymeren für Prothesen (GB-A-1 039 750) oder Gebisshaftmittel (DE-A-2 316 603, US-A-3 926 870, US-A-5 011 868) bekannt. Weiterhin eignen sich gezielt substituierte Acryl- bzw. Methacrylamide als wirksame Haftkomponenten in Dentaladhäsiven, die beispielsweise Gegenstand der US-A-3 660 343 bzw. EP-A-0 394 792 sind oder von S. Kitoh et al. beschrieben wurden (vgl. J. Appl. Polym. Sci. 39 (1990) 103, J. Appl. Polym. Sci. 51 (1994) 2021). Schließlich sind multifunktionelle (Meth)acrylamide aufgrund ihrer im Vergleich zu konventionellen Dimethacrylatvernetzern verbesserten Hydrolysestabilität besonders für wässrig-saure Schmelz-Dentin-Adhäsive (vgl. N. Moszner, F. Zeuner, J. Angermann, U. K. Fischer, V. Rheinberger, Macromol. Mater. Eng. 288 (2003) 621; US-A-6 953 832) und auch für selbsthaftende Komposite (vgl. N. Moszner, U. K. Fischer, J. Angermann, V. Rheinberger, Dent. Mater. 22 (2006) 1157) als Vernetzer besonders geeignet.

N-Alkoxy-N-alkylamide, auch als Weinreb-Amide bezeichnet, sind in der modernen organischen Synthesechemie etablierte Zwischenprodukte z.B. zur Synthese von sterisch anspruchsvollen Ketonen oder als Substrat für enantioselektive Diels-Alder Reaktionen (M. Mentzel, H. M. R. Hoffmann, J. prakt. Chem. 330 (1997) 517). Weinreb-Amide sind einfach zugänglich, z.B. durch Acylierung von N,O-Dimethylhydroxylamin mit entsprechendem Säurechlorid. Außerdem zeichnen sich die Weinreb-Amide durch eine beachtliche Stabilität aus und lassen sich dementsprechend durch übliche Methoden reinigen, z.B. durch Kristallisation, Destillation oder Chromatographie. Als polymerisationsfähiges N,O-funktionalisiertes Carbonsäure-hydroxamid ist vor allem das N-Methoxy-N-methyl-acrylamid bekannt (vgl. U. K. Fischer, N. Moszner, F. Zeuner, V. Rheinberger, Tagungsband der GDCh-Fachgruppentagung Polymers & Coatings, 24.-26.09.2006, Mainz, S. 82). Die Verwendung von polymerisationsfähigen, N,O-funktionalisierten Acrylsäure-hydroxamiden für die Herstellung von Dentalmaterialien ist hingegen nicht beschrieben.

WO 03/035013 beschreibt substituierte Acrylamide als wirksame Haftkomponenten in Dentaladhäsiven.

Der Erfindung liegt die Aufgabe zugrunde, ein Dentalmaterial bereitzustellen, das sich mittels radikalischer Polymerisation sehr schnell aushärten lässt, wobei je nach Anwendungsbereich ein vernetzendes Material (Komposit oder Beschichtung) und/oder auf der Zahnhartsubstanz haftendes Material (Adhäsiv, Komposit oder Beschichtung) gebildet wird, das damit für dentale Zwecke besonders geeignet ist.

Die Aufgabe wird erfindungsgemäß durch ein Dentalmaterial gelöst, das dadurch gekennzeichnet ist, dass es mindestens ein polymerisationsfähiges N,O-funktionalisiertes Acrylsäurehydroxamid der allgemeinen Formel (I) 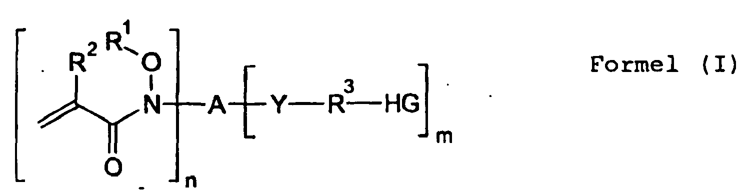 enthält, in der
- A: ein n+m-wertiger linearer oder verzweigter aliphatischer C₁- bis C₅₀-Rest, bei dem die Kohlenstoffkette durch O, S, -CO-O-, CO-NH, O-CO-NH oder NH-CO-NH unterbrochen sein kann, ein n+m-wertiger aromatischer C₆- bis C₁₈-Rest oder einem n+m-wertiger cycloaliphatischer bzw. heterocyclischer C₃- bis C₁₈-Rest ist, wobei die Reste einen oder mehrere Substituenten tragen können,
- Y: entfällt, O, S, eine Ester-, Amid- oder Urethangruppe ist,
- R¹: Wasserstoff, ein aliphatischer C₁- bis C₂₀-Alkyl- oder C₃- bis C₈-Cycloalkylrest ist, der einen oder mehrere Substituenten tragen kann,
- R²: H oder ein C₁- bis C₁₀-Alkylrest ist,
- R³: entfällt oder ein C₁- bis C₁₆-Alkylenrest ist, der durch O unterbrochen sein kann,
- HG: entfällt, -COOH, -P=O(OH)₂; -P=O (OH) (OR⁴) ; -O-P=O (OH)₂, -SO₂OH oder -O-P=O(OH) (OR⁴) ist,
- R⁴: ein C₁- bis C₁₅-Alkylrest, Phenyl- oder Benzyl-Rest ist und
- n: eine Zahl von 1 bis 5 und
- m: eine Zahl von 0 bis 3 ist.

Gegenstand der vorliegenden Erfindung ist ferner ein Amid der allgemeinen Formel (I) zur Verwendung als Dentalmaterial bzw. zur Herstellung eines Dentalmaterials, d.h. eines Materials zur Anwendung als Dentalmaterial.

Unter Dentalmaterial werden erfindungsgemäß Zahnfüllungsmaterialien, Materialien für Inlays oder Onlays, Zahnzemente, Verblendmaterialien für Kronen und Brücken, Materialien für künstliche Zähne oder sonstige Materialien für die prothetische, konservierende und präventive Zahnheilkunde verstanden.

Weitere bevorzugte Ausführungsformen der vorliegenden Erfindung sind in den angefügten abhängigen Ansprüchen offenbart.

Die erfindungsgemäßen polymerisationsfähigen N,O-funktionalisierten Acrylsäure-hydroxamide der allgemeinen Formel (I) stellen Monomere unterschiedlicher Natur und Reaktivität dar, die gegebenenfalls in Mischung mit anderen polymerisationsfähigen Komponenten durch Copolymerisation in Gegenwart von geeigneten radikalischen Initiatoren, thermisch oder bei Einstrahlung von Licht des sichtbaren, UV- oder IR-Bereichs zu mechanisch stabilen Schichten oder Formkörpern führen. Durch Auswahl und Anzahl der polymerisationsfähigen bzw. funktionellen Gruppen lassen sich verschiedene Typen der erfindungsgemäßen polymerisationsfähigen N,O-funktionalisierten Acrylsäure-hydroxamide der allgemeinen Formel (I) unterscheiden:
- n = 1, m = 0: monofunktionelle N,O-funktionalisierte Acrylamide,
- n = 2-5, m = 0: multifunktionelle N,O-funktionalisierte Acrylamide, die als Vernetzer eingesetzt werden können,
- n = 1, m = 1: monofunktionelle N,O-funktionalisierte Acrylamide, die eine saure Haftgruppe tragen,
- n = 2-5, m = 1: vernetzende, multifunktionelle N,O-funktionalisierte Acrylamide, die eine saure Haftgruppe tragen und
- n = 2-5, m = 2-3: vernetzende, multifunktionelle N,O-funktionalisierte Acrylamide, die mehrere saure Haftgruppen besitzen.

Damit lassen sich je nach Auswahl der erfindungsgemäßen polymerisationsfähigen N,O-funktionalisierten Acrylsäure-hydroxamide der allgemeinen Formel (I) Materialien mit unterschiedlichen Eigenschaften und verschiedenen dentalen Anwendungen herstellen.

So eignen sich die multifunktionellen N,O-funktionalisierten Acrylsäureamide (n = 2-5, m = 0) als Vernetzerkomponente besonders für Komposite oder Zemente, wobei die Vernetzungsdichte bzw. damit die mechanischen Eigenschaften, wie z.B. E-Modul, der ausgehärteten Materialien mit zunehmender Funktionalität, d.h. mit zunehmenden n, sich erhöhen bzw. verbessern. Dabei lässt sich auch die Vernetzungsdichte solcher Materialien durch Copolymerisation mit monofunktionellen N,O-funktionalisierten Acrylsäureamiden (n = 1, m = 0) verringern.

Monofunktionelle, N,O-funktionalisierte Acrylamide, die eine saure Haftgruppe tragen (n = 1, m = 1) können als Haftmonomere bei der Herstellung von Adhäsiven eingesetzt werden. Durch die gezielte Zugabe von multifunktionellen, vernetzenden N,O-funktionalisierten Acrylsäureamiden zu diesen Adhäsiven lässt sich deren Aushärtungsgeschwindigkeit erhöhen, wobei sich durch die Vernetzung die Quellbarkeit der Adhäsivschicht verringert und deren Festigkeit zunimmt.

Alternativ lassen sich für solche schnell aushärtenden Adhäsive vor allem die vernetzenden, multifunktionellen N,O-funktionalisierten Acrylsäureamide, die eine saure Haftgruppe tragen (n = 2-5, m = 1) oder sogar die vernetzenden, multifunktionellen N,O-funktionalisierte Acrylsäureamide, die mehrere saure Haftgruppen besitzen (n = 2-5, m = 2-3), einsetzen.

Schließlich eignen sich die N,O-funktionalisierten Acrylsäureamide, die eine oder mehrere saure Haftgruppen tragen und einen oder mehrere N,O-funktionalisierte Acrylsäureamid-Reste enthalten, zur Verwendung als Komponente von dentalen Kompositen, Zementen oder Beschichtungsmaterialien. Die entsprechenden Materialien zeigen dann selbsthaftende Eigenschaften.

Die erfindungsgemäßen N,O-funktionalisierten Acrylamide der allgemeinen Formel (I) lassen sich durch Umsetzung von N,O-funktionalisierten Aminen mit Acylsäurehalogeniden (CH₂=CR²COX, X = Cl oder Br) unter Anwendung der aus der organischen Chemie bekannten Methoden für die Bildung von Amid-Bindungen (vgl, Methoden der Organischen Chemie, HOUBEN-WEYL Bd. E5 1985, Georg Thieme Verlag S. 941ff.) herstellen:

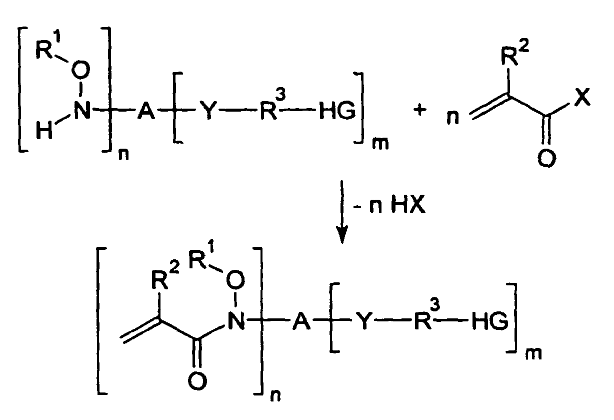

Dabei werden bevorzugt die entsprechenden Säurechloride in Gegenwart äquimolarer Mengen an Hilfsbase, z.B. Triethylamin oder Pyridin verwendet (In dem folgenden speziellen Beispiel ist R¹ = CH₃, R² = H, A = C₆H₁₂, n = 2; Y, R³ und HG entfallen.):

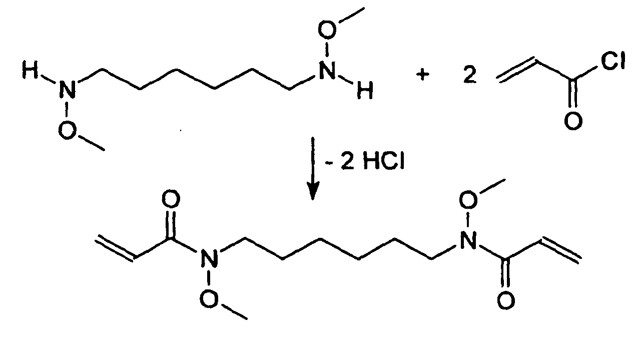

Dabei lassen sich die hierbei eingesetzten sekundären N-Methoxyamine über die 2-Nitrobenzolsulfonyl-Schutzgruppe analog zur Synthese von sekundären Aminen (vgl. W. Kurosawa, T. Kan, T. Fukuyama, Org. Synth. 79 (2002) 186) herstellen. Beispielsweise ist das vorstehende 1,6-Di(N-methoxyamino)hexan durch Umsetzung von 1,6-Dibromhexan mit N-Methoxy-2-nitrobenzolsulfamid und Entschützung des gebildeten Sulfamides mit Thiophenol (Ph-SH) zugänglich. Dabei kann das benötigte Methoxy-2-nitrobenzolsulfamid einfach durch Umsetzung von O-Methylhydroxylaminhydrochlorid mit 2-Nitrobenzolsulfonylchlorid synthetisiert werden:

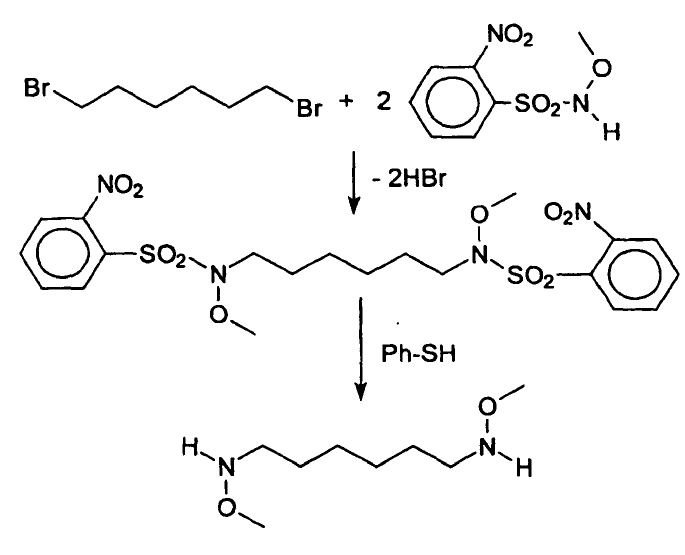

Analog lassen sich auch andere Hydroxylamin-Derivate der allgemeinen Struktur R¹-O-NH₂ umsetzen. Dabei können diese Hydroxylamin-Derivate durch Reaktion der entsprechenden Alkohole R¹-OH mit Triphenylphosphin ((Ph)₃P), N-Hydroxyphthalimid und Diisopropyl-azodicarboxylat (DIAD) und anschliessende Hydrazinolyse des gebildeten Phthalimido-Derivates hergestellt werden (vgl. E. Grochowski, J. Jurczak, Synthesis 1976, 682; S. Su, J. R. Giguere, S. E. Schaus, J. A. Porco, Tetrahedron 60 (2004) 8645):

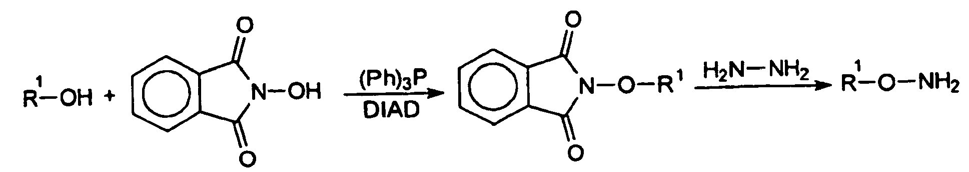

Werden erfindungsgemäße N,O-funktionalisierte Acrylamide der allgemeinen Formel (I) hergestellt, die ein oder mehrere Säuregruppen HG tragen, so ist ebenfalls die Anwendung der entsprechend üblichen Schutzgruppentechnik notwendig. Dementsprechend ist es vorteilhaft, die Schutzgruppe der Säurefunktion erst im letzten Syntheseschritt abzuspalten. Beispielsweise lassen sich N,0-funktionalisierte Acrylamide mit einer Phosphonsäurefunktion (z.B. R¹ = CH₃, R² = H, HG = -P=O(OH)₂, n und m = 1, A = Alkylen, R³ und Y entfallen) so herstellen, dass zunächst ein α,ω-Halogenalkylenphosphonsäuredi-tert.-butylester (tert.-Butylgruppe = Schutzgruppe, X = Halogen wie Cl oder Br) mit N-Methoxy-2-nitrobenzolsulfamid umgesetzt wird. Durch Entschützung des gebildeten Sulfamides und nachfolgender Umsetzung der N-Methoxyamino-Gruppe mit Acrylsäurechlorid bildet sich das N,0-funktionalisierte Acrylsäureamid, dessen Phosphonsäuregruppe nach Abspaltung der beiden tert.-Butyl-Schutzgruppen mit Trifluoressigsäure (TFA) freigesetzt wird.

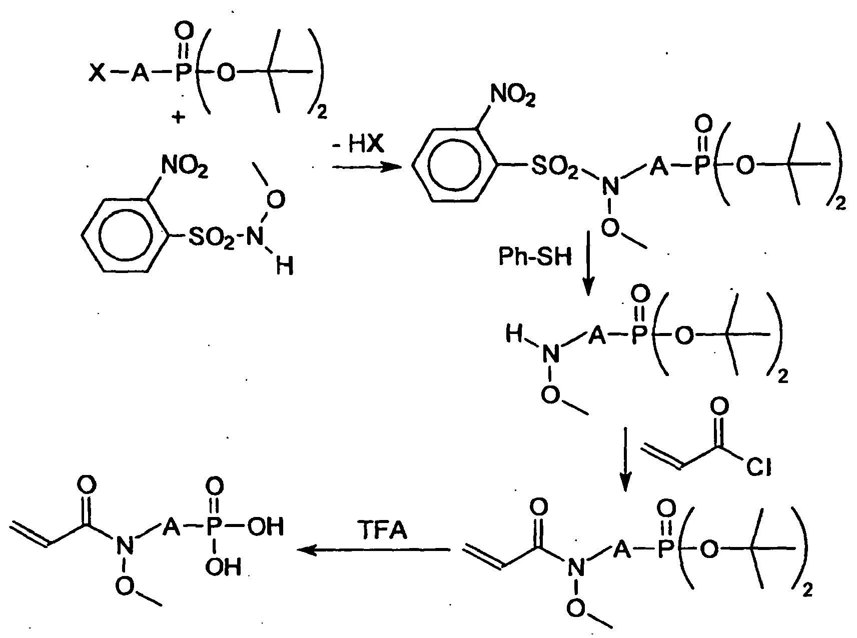

Bei einer bevorzugten Ausführungsform der Erfindung sind der eine oder die mehreren Substituenten an dem Rest A ausgewählt sind aus der Gruppe bestehend aus C₁- bis C₅-Alkylgruppen, Cl, Br und OH, insbesondere C₁- bis C₃-Alkylgruppen und OH. Vorzugsweise sind der eine oder die mehreren Substituenten an dem Rest R¹ ausgewählt aus der Gruppe bestehend aus Cl, Br und OH.

Ein bevorzugtes erfindungsgemäßes Dentalmaterial auf Basis eines N,O-funktionalisierten Acrylsäureamids der allgemeinen Formel (I) ist ein solches, bei dem
- A: ein n+m-wertiger linearer oder verzweigter aliphatischer C₁- bis C₃₀-Rest, z.B. ein C₁- bis C₁₅-Rest oder ein C₁- bis C₁₀-Rest, bei dem die Kohlenstoffkette durch O, -CO-O- oder O-CO-NH unterbrochen sein kann, ein n+m-wertiger aromatischer C₆- bis C₁₀-Rest, oder ein n+m-wertiger cycloaliphatischer C₃- bis C₁₈-Rest ist, wobei die Reste einen oder mehrere Substituenten tragen können,
- Y: entfällt, O, eine Ester- oder Urethangruppe ist,
- R¹: ein aliphatischer C₁- bis C₈-Alkyl- oder Cycloalkylrest ist, z.B. ein C₁- bis C₄-Alkyl- oder Cyclohexylrest,
- R²: H oder CH₃ ist,
- R³: entfällt oder ein C₁- bis C₁₂-Alkylenrest, der durch O unterbrochen sein kann, ist, z.B. ein C₆- bis C₁₂-Alkylenrest,
- HG: entfällt, -COOH, -P=O(OH)₂, -O-P-O(OH)₂, -SO₂OH oder -O-P=O(OH) (OR⁴) ist,
- R⁴: ein C₁- bis C₄-Alkylrest ist, z.B. Methyl, Ethyl, Propyl oder Butyl,
- n: eine Zahl von 1 bis 3 ist und
- m: eine Zahl von 0 bis 2 ist.

Ein weiteres bevorzugtes erfindungsgemäßes Dentalmaterial auf Basis eines N,0-funktionalisierten Acrylsäureamids der allgemeinen Formel (I) ist ein solches, bei dem
- A: ein n+m-wertiger linearer oder verzweigter aliphatischer C₁- bis C₁₂-Rest, z.B. C₁- bis C₁₀-Alkyl oder C₁- bis C₁₂-Alkylen, ein n+m-wertiger cycloaliphatischer C₆- bis C₁₂-Rest oder ein n+m-wertiger aromatischer C₆- bis C₁₀-Rest ist,
- Y: entfällt oder eine Ester-Gruppe ist,
- R¹: C₁- bis C₄-Alkyl ist,
- R²: H oder CH₃ ist,
- R³: entfällt oder C₄- bis C₈-Alkylen-Rest ist, der durch O unterbrochen sein kann,
- HG: entfällt, -COOH, -P=O(OH)₂, -OP=O(OH)₂, -OP=O(OH) (OC₂H₅) oder -SO₂OH ist,
- n: 1, 2 oder 3 und
- m: 0 oder 1 ist.

Insbesondere ist ein erfindungsgemäßes Dentalmaterial auf Basis eines N,O-funktionalisierten Acrylsäureamids der allgemeinen Formel (I) ein solches, bei dem
- A: ein n+m-wertiger linearer oder verzweigter aliphatischer C₁- bis C₅-Rest wie Ethyl, Propyl, Butyl, Ethylen, Propylen, Butylen, Pentylen oder Pentantriyl, ein n+m-wertiger cycloaliphatischer C₆-Rest wie Cyclohexyl, Cyclohexylen, Cyclohexantriyl, oder ein n+m-wertiger aromatischer C₆-Rest wie Phenyl oder Benzoltriyl ist,
- Y: entfällt oder -COO- ist,
- R¹: Methyl, Ethyl oder Propyl ist,
- R²: H oder CH₃ ist,
- R³: entfällt oder Pentylen ist,
- HG: entfällt, -COOH, -P=O(OH)₂, -OP=O(OH)₂, -OP=O(OH) (OC₂H₅) oder -SO₂OH ist,
- n: 1, 2 oder 3 und
- m: 0 oder 1 ist.

Z. B. kann in einem bevorzugten erfindungsgemäßen N,O-funktionalisierten Acrylsäureamid der allgemeinen Formel (I) A unsubstituiertes C₆-Alkylen, R¹ Methyl, R² H und n 2 bedeuten, wobei m = 0 ist. Alternativ kann A vorzugsweise unsubstituiertes C₁₀-Alkylen, R¹ Methyl, R² H, n und m jeweils 1 und HG -OP=O(OH)₂ bedeuten, wobei die Reste Y und R³ entfallen.

Spezielle Beispiele für die erfindungsgemäßen N,O-funktionalisierten Acrylsäureamide der allgemeinen Formel (I) sind u. a.:

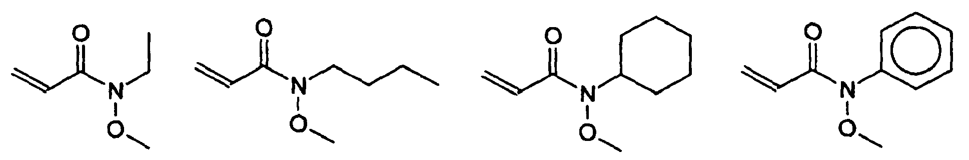

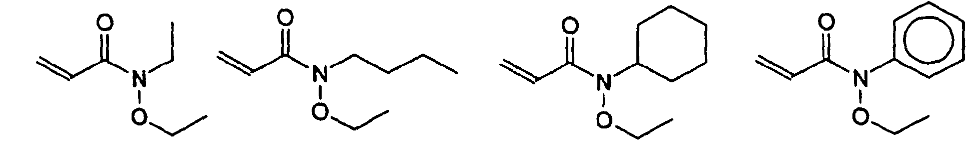

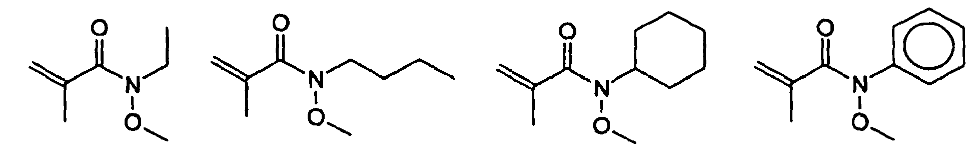

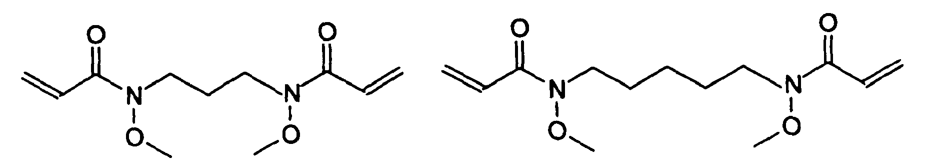

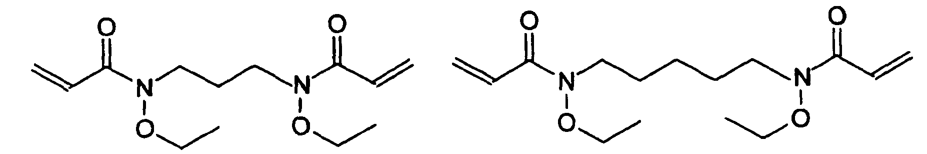

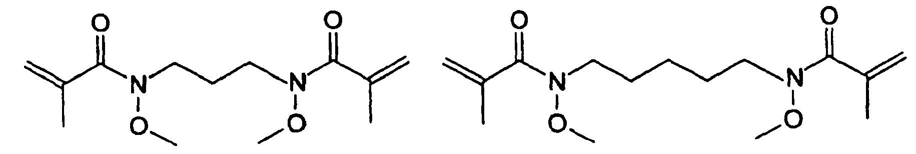

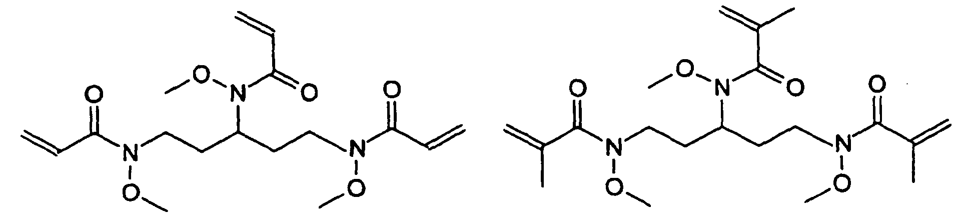

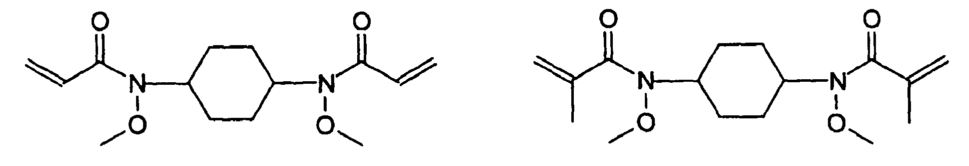

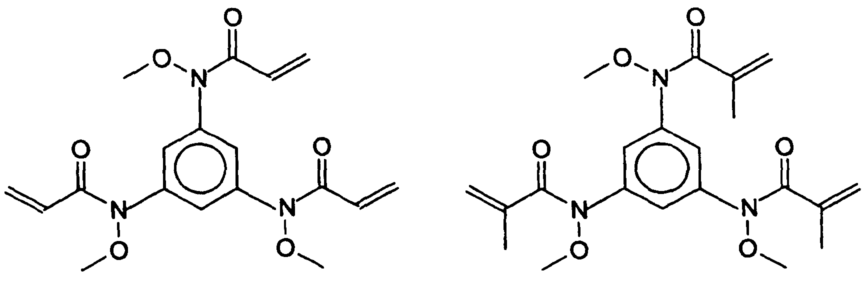

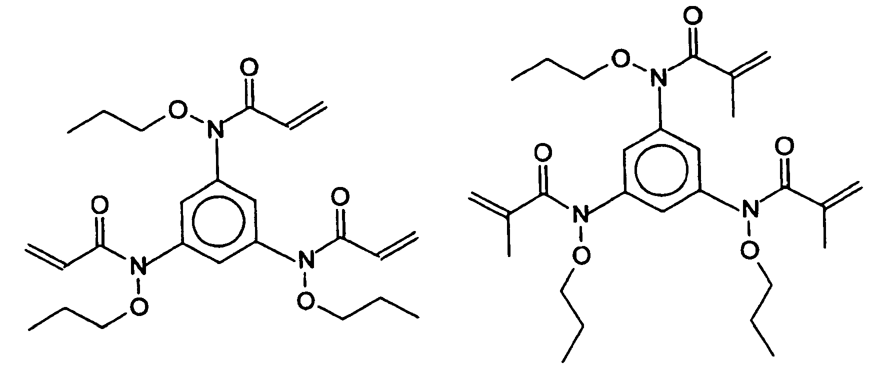

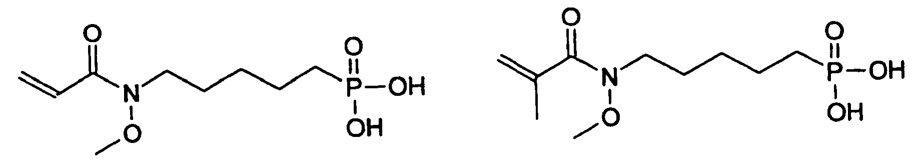

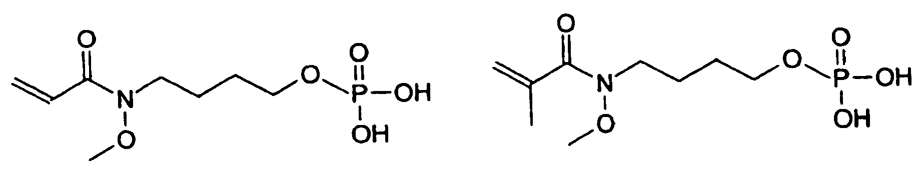

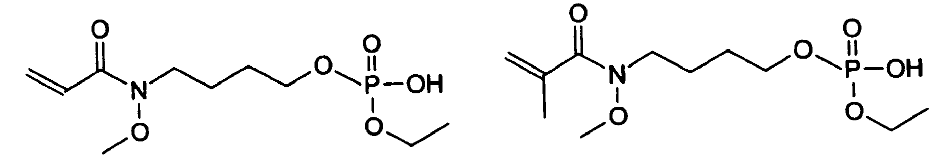

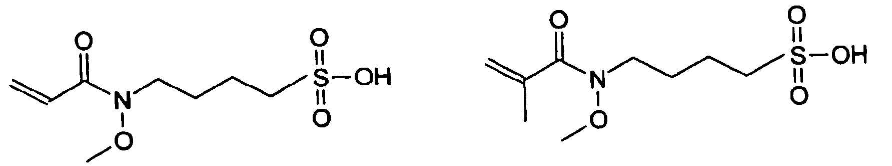

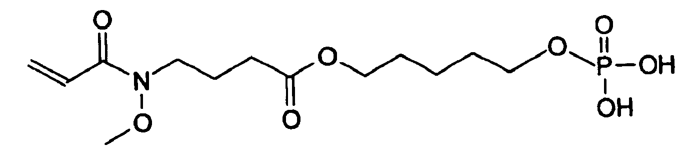

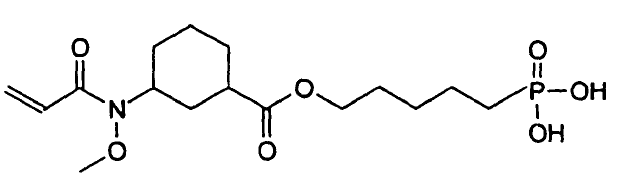

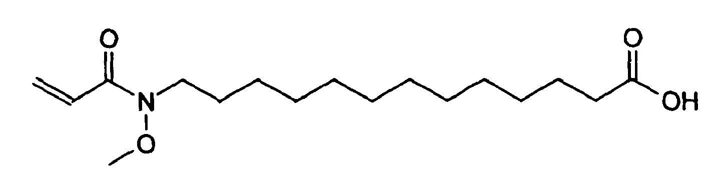

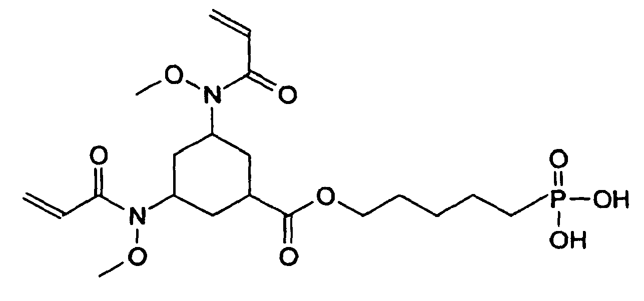

Zur Herstellung der erfindungsgemäßen Dentalmaterialien werden die N,O-funktionalisierten Acrylsäureamide allein, ihre Mischungen untereinander oder ihre Mischungen mit anderen Verdünner- oder Vernetzermonomeren durch radikalische Polymerisation ausgehärtet. Vor der Polymerisation lassen sich neben dem Initiator evtl. geeignete Lösungsmittel oder weitere Additive, wie Füllstoffe, Stabilisatoren oder andere Adhäsivmonomere zusetzen.

Als geeignete radikalisch polymerisierbare Verdünnermonomere kommen Mono(meth)acrylamide und/oder Mono(meth)acrylate, z.B. Acrylamid, Methacrylamid, N-Ethylacrylamid, Methyl-, Ethyl-, Butyl-, Benzyl-, Furfuryl- oder Phenyl(meth)acrylat in Frage. Als weitere Vernetzermonomere lassen sich bekannte multifunktionelle Acrylate bzw. Methacrylate, wie z.B. Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- oder Tetraethylenglycoldi(meth)acrylat, Decandioldi(meth)acrylat, Trimethylolpropantri(meth)-acrylat, Pentaerythrittetra(meth)acrylat sowie Butandioldi(meth)acrylat, 1,10-Decandioldi(meth)acrylat oder 1,12-Dodecandioldi(meth)acrylat einsetzen.

Die Aushärtung der Zusammensetzungen auf der Basis der N,O-funktionalisierten Acrylamide erfolgt nach Zugabe geeigneter Initiatoren durch thermische, photochemische oder redoxinduzierte radikalische Polymerisation. Bevorzugte Beispiele für thermische Initiatoren sind die bekannten Peroxide, wie z.B. Dibenzoylperoxid, Dilaurylperoxid, tert.-Butylperoctoat oder tert.-Butylperbenzoat sowie weiter Azobisisobutyroethylester, Azobisisobutyronitril, Azobis-(2-methylpropionamidin)dihydrochlorid, Benzpinakol oder 2,2-Dimethylbenzpinakol.

Beispiele für geeignete Photoinitiatoren sind Dibenzoyldiethylgermanium oder Dibenzoyldimethylgermanium, Benzophenon, Benzoin sowie deren Derivate oder α-Diketone oder deren Derivate wie 9,10-Phenanthrenchinon, Diacetyl oder 4,4-Dichlorbenzil. Bevorzugt werden Campherchinon und 2,2-Dimethoxy-2-phenyl-acetophenon und besonders bevorzugt α-Diketone in Kombination mit Aminen als Reduktionsmittel, wie z.B. 4-(N,N-Dimethylamino)-benzoesäureester, N,N-Dimethylaminoethylmethacrylat, N,N-Dimethyl-sym.-xylidin oder Triethanolamin, eingesetzt. Darüber hinaus sind auch Acylphosphine, wie z.B. 2,4,6-Trimethylbenzoyldiphenyl- oder Bis(2,6-dichlorbenzoyl)-4-N-propylphenylphosphinoxid besonders geeignet.

Als Initiatoren für eine bei Raumtemperatur durchgeführte Polymerisation werden Redox-Initiatorkombinationen, wie z.B. Kombinationen von Benzoyl- oder Laurylperoxid mit N,N-Dimethyl-sym.-xylidin oder N,N-Dimethyl-p-toluidin, verwendet.

Die N,O-funktionalisierten Acrylsäureamidvernetzer eignen sich besonders als Vernetzungskomponente von Lösungen bekannter stark saurer Adhäsivmonomere. Dazu gehören solche Phosphorsäureestermethacrylate, wie z.B. 2-(Methacryloyloxy)ethyldihydrogenphosphat, Di-[2-(methacryloyloxy)ethyl]-hydrogenphosphat oder Dipentaerythritolpentamethacryloyloxydihydrogenphosphat (vgl. N. Nakabayashi, P. D. Pashley, Hybridization of dental hard tissues, Quintess. Publ. Tokyo etc. 1998, 9 ff) und besonders eignen sich hydrolysestabile Acrylphosphonsäuren, wie z.B. 2-[3-(Dihydroxyphosphoryl)-oxa-propyl]acrylsäureethylester oder 1,2-Bis[1-dihydroxyphosphoryl)-1-[2-methylen-3-ylpropansäureethylester)oxy]methyl]-benzol, die in der DE-A-197 46 708 beschrieben sind.

Als Lösungsmittel für die N,0-funktionalisierten Acrylsäureamide können vor allem polare Lösungsmittel, wie Wasser, Ethanol, Aceton, Acetonitril oder Mischungen aus diesen Lösungsmitteln eingesetzt werden.

Gegebenenfalls können die erfindungsgemäß eingesetzten Zusammensetzungen weitere Additive enthalten, wie z.B. Farbmittel (Pigmente oder Farbstoffe), Stabilisatoren, Aromastoffe, mikrobiocide Wirkstoffe, Weichmacher oder UV-Absorber.

Weiterhin können die erfindungsgemäß eingesetzten Zusammensetzungen zur Verbesserung der mechanischen Eigenschaften mit anorganischen Partikeln oder Fasern gefüllt werden. Bevorzugte anorganische partikuläre Füllstoffe sind amorphe kugelförmige Materialien auf der Basis von Oxiden, wie ZrO₂ und TiO₂ bzw. Mischoxiden aus SiO₂, ZrO₂ und/oder TiO₂, nanopartikuläre oder mikrofeine Füllstoffe, wie pyrogene Kieselsäure oder Fällungskieselsäure sowie Makro- oder Minifüllstoffe, wie Quarz-, Glaskeramik- oder Glaspulver mit einer durchschnittlichen Teilchengrösse von 0,01 bis 5 µm sowie röntgenopake Füllstoffe, wie Ytterbiumtrifluorid.

Eine erfindungsgemäß bevorzugt eingesetzte Zusammensetzung enthält, jeweils bezogen auf die Gesamtmasse des Materials:
(a) 1 bis 95 Gew.-% Acrylamid der allgemeinen Formel (I),
(b) 0 bis 70 Gew.-% Verdünnermonomer,
(c) 0 bis 70 Gew.-% Vernetzermonomer,
(d) 0,1 bis 5,0 Gew.-% Polymerisationsinitiator und
(e) 0 bis 80 Gew.-% Füllstoff,
(f) 0 bis 70 Gew.-% Lösungsmittel,
(g) 0 bis 70 Gew.-% Adhäsivmonomer.

Dabei werden unter Verdünnermonomer und Vernetzermonomer solche Monomere verstanden, die nicht unter Formel (I) fallen. Unter Adhäsivmonomer werden solche Monomere verstanden, die eine oder mehrere saure Haftgruppen aufweisen und unter die Formel (I) fallen oder auch nicht.

Eine erfindungsgemäß besonders bevorzugt eingesetzte Zusammensetzung enthält, jeweils bezogen auf die Gesamtmasse des Materials:
(a) 5 bis 70 Gew.-% Acrylamid der allgemeinen Formel (I),
(b) 0 bis 40 Gew.-% Verdünnermonomer,
(c) 0 bis 40 Gew.-% Vernetzermonomer,
(d) 0,2 bis 2,0 Gew.-% Polymerisationsinitiator und
(e) 0 bis 50 Gew.-% Füllstoff,
(f) 0 bis 50 Gew.-% Lösungsmittel,
(g) 0 bis 50 Gew.-% Adhäsivmonomer.

Eine erfindungsgemäß bevorzugt als dentales Adhäsiv einsetzbare Zusammensetzung enthält, jeweils bezogen auf die Gesamtmasse des Materials:
(a) 5 bis 40 Gew.-%, insbesondere 10 bis 40 Gew.-% Acrylamid der allgemeinen Formel (I),
(b) 0 bis 40 Gew.-% Verdünnermonomer,
(c) 0 bis 70 Gew.-%, insbesondere 50 bis 70 Gew.-% Vernetzermonomer,
(d) 0,2 bis 2,0 Gew.-% Polymerisationsinitiator,
(e) 2 bis 50 Gew.-%, insbesondere 20 bis 50 Gew.-% Lösungsmittel,
(f) 5 bis 40 Gew.-%, insbesondere 10 bis 40 Gew.-% Adhäsivmonomer,
(g) 0 bis 20 Gew.-% Nanofüller (Füllstoff mit einer Primärteilchengröße von < 50 nm).

Eine erfindungsgemäß bevorzugt als dentaler Zement einsetzbare Zusammensetzung enthält, jeweils bezogen auf die Gesamtmasse des Materials:
(a) 5 bis 20 Gew.-% Acrylamid der allgemeinen Formel (I),
(b) 0 bis 20 Gew.-% Verdünnermonomer,
(c) 0 bis 20 Gew.-% Vernetzermonomer,
(d) 0,2 bis 2,0 Gew.-% Polymerisationsinitiator,
(e) 5 bis 60 Gew.-% Füllstoff,
(f) 2 bis 20 Gew.-% Adhäsivmonomer.

Eine erfindungsgemäß bevorzugt als dentales Füllungsmaterial einsetzbare Zusammensetzung enthält, jeweils bezogen auf die Gesamtmasse des Materials:
(a) 5 bis 20 Gew.-% Acrylamid der allgemeinen Formel (I),
(b) 0 bis 30 Gew.-% Verdünnermonomer,
(c) 0 bis 30 Gew.-% Vernetzermonomer,
(d) 0,2 bis 2,0 Gew.-% Polymerisationsinitiator,
(e) 10 bis 80 Gew.-%, insbesondere 50 bis 80 Gew.-% Füllstoff.

Eine erfindungsgemäß bevorzugt als dentales Beschichtungssmaterial einsetzbare Zusammensetzung enthält, jeweils bezogen auf die Gesamtmasse des Materials:
(a) 5 bis 40 Gew.-% Acrylamid der allgemeinen Formel (I),
(b) 0 bis 50 Gew.-% Verdünnermonomer,
(c) 0 bis 50 Gew.-% Vernetzermonomer,
(d) 0,2 bis 2,0 Gew.-% Polymerisationsinitiator,
(e) 2 bis 50 Gew.-% Lösungsmittel,
(f) 0 bis 20 Gew.-% Nanofüller (Füllstoff mit einer Primärteilchengröße von < 50 nm).

Die Erfindung wird im Folgenden anhand von Beispielen näher erläutert.

### Beispiel 1: Synthese von 1,6-Bis(N-acryloyl-N-methoxyamino) hexan

### 1. Stufe: N-Methoxy-2-nitrobenzolsulfonamid (1):

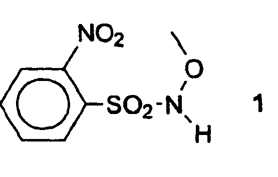

Zu einer Lösung von O-Methylhydroxylamin-hydrochlorid (11,7 g, 141 mmol) in Pyridin (100 ml) wurde 2-Nitrobenzolsulfonylchlorid (28,8 g, 130 mmol) innerhalb von 30 min unter Rühren und Wasserkühlung zugegeben. Nach der Zugabe wurde das Gemisch noch 30 min bei Raumtemperatur (RT) gerührt, mit Eis (200-300 g) versetzt und mit konz. HCl bis zum pH-Wert 2-3 angesäuert. Die noch kalte Suspension wurde abgesaugt, der Niederschlag mit Eiswasser sorgfältig gewaschen und getrocknet. Man erhielt 26,4 g (87%) des Sulfamids **1** als einen gelblichen Feststoff vom Schmp. 133-136 °C. ¹H-NMR (DMSO-D₆, 300 MHz): δ = 3,67 (s, 3 H), 7,92-8,06 (m, 4 H), 11,05 (s, 1 H) ppm.

### 2. Stufe: N,N'-Dimethoxy-2,2'-dinitrohexamethylendi(benzolsulfamid) (2) :

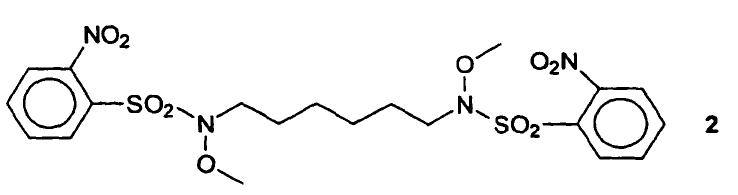

Ein Gemisch aus dem Sulfamid **1** (24,1 g, 104 mmol), 1,6-Dibromhexan (12,2 g, 5,0 mmol) und K₂CO₃ (17,0 g, 123 mmol) in DMF (100 ml) wurde bei 70-80 °C 3 h intensiv gerührt, abgekühlt und mit etwa dem 5-fachen Volumen Wasser versetzt. Nach der Verdünnung mit Wasser fiel das Rohprodukt als Niederschlag aus. Es wurde abgesaugt, mit viel Wasser gewaschen und in CH₂Cl₂ (ca. 500 ml) gelöst. Die Lösung wurde über wasserfreiem MgSO₄ getrocknet und im Vakuum bis etwa auf ein Viertel des Volumens eingeengt. Der Rückstand wurde mit etwa dem gleichen Volumen Hexan versetzt, nach 1 h Stehen im Kühlschrank abgesaugt, mit einer 1:1-Mischung aus Methylenchlorid und Hexan gewaschen und getrocknet. Man erhielt 26,1 g (96%) des Sulfonamids 2 als fast farblosen Feststoff vom Schmp. 145-146 °C. ¹H-NMR (DMSO-D₆, 300 MHz): δ = 1,37 (m_{c}, 4 H), 1,59 (m_{c}, 4 H), 2,99 (t, *J* = 7 Hz, 4 H), 3,77 (s, 6 H), 7,86-7,92 (m, 2 H), 7,97-8,02 (m, 6 H) ppm.

### 3. Stufe: 1,6-Di(N-methoxyamino)hexan (3):

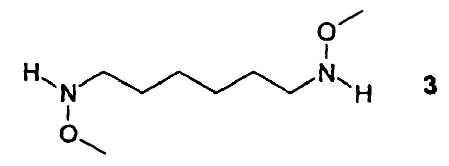

Ein Gemisch aus dem Sulfamid 2 (28,1 g, 51,4 mmol), K₂CO₃ (43 g, 312 mmol) und Thiophenol (22,6 g, 206 mmol) in DMF (150 ml) wurde 2-6 h bei 40-45 °C intensiv gerührt (DC-Kontrolle). Zur Aufarbeitung wurde das Gemisch mit dem etwa 2-fachen Volumen Wasser und Ether verdünnt. Die abgekühlte Lösung wurde sehr vorsichtig mit konz. HCl angesäuert (CO₂-Entwicklung, Thiophenol) und die saure wässrige Phase wurde abgetrennt. Die organische Phase wurde noch mit 5%iger HCl (2 × 100 ml) gewaschen. Die vereinigten sauren Phasen wurden mit Ether (2 × 100 ml) gewaschen, mit Eiswasser abgekühlt und sehr vorsichtig mit festem KOH stark alkalisch gemacht (heftige Wärmeentwicklung). Das Produkt schied sich als ein gelb-oranges Ö1 ab. Dieses wurde mit Ether (3 × 50 ml) extrahiert, die Lösung über K₂CO₃ getrocknet und im Vakuum eingeengt. Man erhielt 8,48 g (89%) des Diamins 3. Zur Endreinigung wurde dieses Diamin im Vakuum fast ohne Verluste destilliert, Sdp. 95-97 °C (0,5 Torr). ¹H-NMR (CDCl₃, 300 MHz): δ = 1,33 (m_{c}, 4 H), 1,47 (m_{c}, 4 H), 2,87 (t, *J* = 7 Hz, 4 H), 3,50 (s, 6 H), 5,49 (br. s, 2 H) ppm.

### 4. Stufe: 1,6-Bis(N-acryloyl-N-methoxyamino)hexan (4):

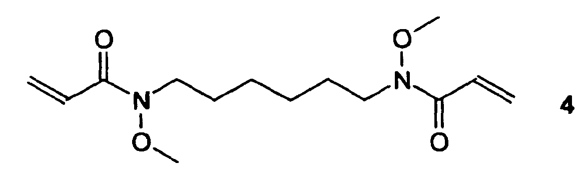

Zu einer gerührten Suspension des Bis-methoxyamins **3** (881 mg, 5 mmol), Na₂CO₃ (2,65 g, 25 mmol) und BHT (= 2,6-tert.-butyl-4-methylphenol) (10 mg) in wasserfreiem Diethylether (100 ml) wurde unter Eiskühlung Acrylsäurechlorid (996 mg, 11 mmol) innerhalb von 5 min zugetropft. Man liess dann die Reaktionsmischung auf Raumtemperatur erwärmen und rührte weitere 16 h. Nachdem das Bis-methoxyamin **3** vollständig verbraucht war, wurde Wasser (20 ml) zugegeben, die organische Phase abgetrennt und die wässrige Phase mit Diethylether (3 × 20 ml) extrahiert. Die organischen Phasen wurden vereinigt, das Lösungsmittel im Vakuum entfernt und das Rohprodukt säulenchromatographisch gereinigt (Kieselgel, Elutionsmittel Chloroform/Ethylacetat 3:1). Es wurden 1,1 g (79%) 1,6-Bis(N-acryloyl-N-methoxyamino)hexan **4** erhalten. ¹H-NMR (300 MHz, CDCl₃) : δ = 1,31-1,36 (m, 4 H), 1, 59-1, 69 (m, 4 H), 3,65 (t, J = 7,2 Hz, 4 H), 3,68 (s, 6 H), 5,74 (dd, J = 10,3, 2,1 Hz, 2 H), 6, 41 (dd, J = 17,1, 2, 1 Hz, 2 H), 6, 71 (dd, J = 17, 1, 10,3 Hz, 2 H) ppm. ¹³C-NMR (75 MHz, CDCl₃) : δ = 26,4 (2 CH₂), 26,9 (2 CH₂), 44,9 (2 CH₂), 62, 1 (2 CH₃), 126,2 (CH), 128,9 (CH₂), 166, 1 (2 C) ppm.

### Beispiel 2: Synthese von [10-(N-Acryloyl-N-methoxyamino) decyl]phosphorsäure (5)

### 1. Stufe: 10-Hydroxydecyl-di-tert-butylphosphat (6):

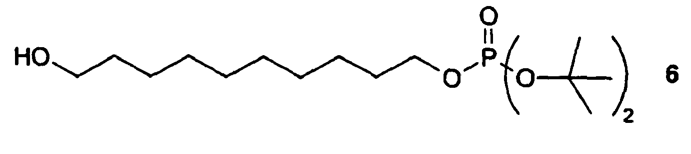

Unter Stickstoff wurde zu einer Mischung aus 1,10-Decandiol (34,9 g, 200 mmol) und Natriumhydrid (8,1 g einer 60%-igen Suspension in Mineralöl, 200 mmol) unter Rühren wasserfreies THF (400 ml) zugegeben. Das Reaktionsgemisch wurde 18 h am Rückfluss erhitzt. Nach Zugabe von di-tert-Butylchlorphosphat (27 g, 118 mmol), das nach der Literatur (vgl. H. Goldwhite, B. C. Saunders, J. Chem. Soc. 1957, 2409-2412; T. Gajda, A. Zwierzak, Synthesis 1976, 243-244) aus PC1₃ und *t*e*rt*-Butanol in 2 Stufen zugänglich war, wurde weitere 1,5 h unter Rückfluss erhitzt und dann das Lösungsmittel im Vakuum entfernt. Der Rückstand wurde mit einer Mischung aus *tert*-Butylmethylether (MTBE, 400 ml) und Wasser (100 ml) versetzt. Nach Abtrennen der organischen Phase wurde diese mit 10%iger NaCl-Lösung und mit gesättigter NaCl-Lösung gewaschen. Dann wurde über wasserfreiem Magnesiumsulfat getrocknet und das Lösungsmittel entfernt. Der Rückstand wurde in etwas siedendem MTBE gelöst. Durch Zugabe der 5-fachen Menge Pentan konnte nicht umgesetztes Decandiol auskristallisiert werden. Nach Filtration wurde das Rohprodukt säulenchromatographisch gereinigt (Kieselgel, Elutionsmittel: Chloroform/MTBE 8:1 bis 3:1). Es wurden 19,3 g (45%) 10-Hydroxydecyl-di-tert-butylphosphat **6** als farbloses, hochviskoses Öl erhalten.

### 2. Stufe: 10-(Methansulfonyloxy)decyl-di-tert-butylphosphat (7) :

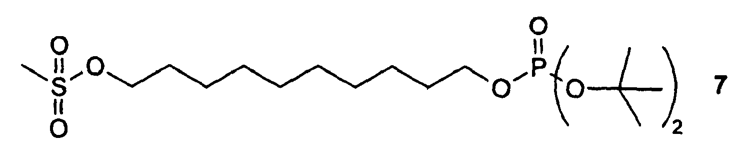

Zu einer Lösung von 10-Hydroxydecyl-di-tert-butylphosphat **6** (12,6 g, 34,4 mmol), Triethylamin (5,5 ml, 39,5 mmol) und DMAP (= 4-(Dimethylamino)-pyridin) (220 mg, 1,8 mmol) in wasserfreiem Methylenchlorid (35 ml) wurde unter Rühren frisch destilliertes Mesylchlorid (4,5 g, 39,3 mmol) über einen Zeitraum von 1 h so zugetropft, dass die Temperatur der Reaktionsmischung zwischen -5 and 0 °C blieb. Danach wurde auf Raumtemperatur erwärmt, noch 1 h gerührt, das Lösungsmittel unter reduziertem Druck entfernt und der Rückstand mit einer Mischung aus MTBE (= Methyl-tert.-butylether) (100 ml) und Wasser (50 ml) versetzt. Diese Mischung wurde mit 10%iger H₂SO₄ auf einen pH-Wert von ~5 eingestellt. Die wässrige Phase wurde mit MTBE (3 × 30 ml) extrahiert. Die vereinigten organischen Phasen wurden mit Wasser (30 ml) und gesättigter NaCl-Lösung (2 × 50 ml) gewaschen und über wasserfreiem Magnesiumsulfat getrocknet. Das Abdestillieren des Lösungsmittels ergab 15,2 g 10-(Methansulfonyloxy)decyl-di-tert-butylphosphat 7 als leicht gelbliches Ö1. ¹H-NMR (CDCl₃, 250 MHz): δ = 1,25-1,45 (m, 12 H), 1,47 (s, 18 H), 1,58-1,80 (m, 4 H), 3,00 (s, 3 H), 3,93 (q, J = 6,6 Hz, 2 H), 4,22 (t, J = 6,6 Hz, 2 H) ppm.

### 3. Stufe: 10-Methoxyaminodecyl-di-tert-butylphosphat (8):

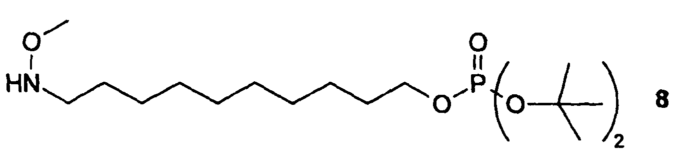

Zu einer Suspension von wasserfreiem NaI (6,85 g, 45,7 mmol) in trockenem Aceton (30 ml) wurde unter Rühren das Mesylat 7 (15,2 g, 34,2) in Aceton (5 ml) zugegeben. Das Gemisch wurde 24 h bei Raumtemperatur gerührt. Dann wurde das Lösungsmittel entfernt und der Rückstand wurde in einer Mischung aus MTBE (100 ml), Wasser (50 ml) und 100 mg Na₂S₂O₄ verteilt. Man trennte die organische Phase ab und extrahierte die wässrige Phase mit MTBE (3 × 30 ml). Die vereinigten organischen Phasen wurden mit Wasser (30 ml) und gesättigter NaCl-Lösung (2 × 50 ml) gewaschen, über wasserfreiem Magnesiumsulfat getrocknet. Anschliessend wurde das Lösungsmittel entfernt. Das so erhaltene leicht gelbe flüssige Iodid (16,3 g, 34 mmol) (¹H-NMR (CDCl3, 250 MHz): δ = 1,25-1,46 (m, 12 H), 1,48 (s, 18 H), 1,64 (tt, *J* = 6,8, 6,6 Hz, 2 H), 1,81 (tt, *J* = 7,0, 6,8 Hz, 2 H), 3,18 (t, J = 7,0 Hz, 2 H), 3,93 (q, J = 6,6 Hz, 2 H) ppm) wurde in wasserfreiem THF (14 ml) gelöst und unter Rühren zu einer Suspension von Methoxyamin (14,0 g, 297 mmol) und NaH (1,36 g einer 60 %-igen Suspension in Mineralöl, 34 mmol) in THF (20 ml) zugegeben. Die Reaktionsmischung wurde unter Rückfluss 16 h erhitzt. Nach Abdampfen des Lösungsmittels im Vakuum wurde der Rückstand mit trockenem Hexan versetzt und 2 h gerührt. Es wurde über eine dünne Schicht aus Celite^{®} filtriert und das Lösungsmittel entfernt. Das Rohprodukt (12,4 g) wurde durch Flash-Chromatographie (Kieselgel (450 ml), Elutionsmittel Chloroform/MTBE/Ethylacetat, 4:1:0 bis 4:1:1) gereinigt, wobei 8,58 g (64%) an 10-Methoxyaminodecyl-di-tert-butylphosphat **8** erhalten wurden. ¹H-NMR (CDCl₃, 300 MHz): δ = 1,22-1,40 (m, 12 H), 1,47 (s, 18 H), 1,45-1,55 (m, 2 H), 1,64 (tt, J = 6,8, 6,6 Hz, 2 H), 2,90 (t, J = 7,3 Hz, 2 H), 3,53 (s, 3 H), 3,93 (q, J = 6,6 Hz, 2 H), 5,53 (bs, 1 H) ppm. ¹³C-NMR (CDCl₃, 75,5 MHz): δ = 25, 6 (CH₂), 27, 2 (CH₂), 27, 3 (CH₂), 29, 2 (CH₂), 29, 4 (CH₂), 29, 5 (CH₂), 29,8 (CH₃), 29, 9 (CH₃), 30, 2 (CH₂), 30, 3 (CH₂), 51, 9 (CH₂), 62,8 (CH₃), 66,8 (CH₂), 66,9 (CH₂), 81,8 (C), 81,9 (C) ppm.

### 4. Stufe: [10-(N-Acryloyl-N-methoxyamino)decyl]phosphorsäure-di-tert-butylester (9):

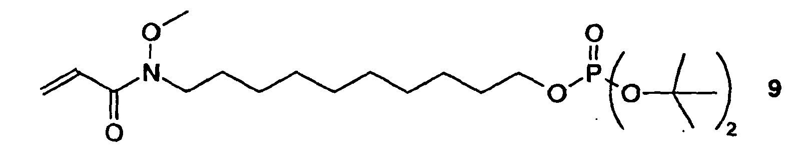

Zu einer Lösung von Methoxyamin **8** (3,96 g, 10 mmol), Triethylamin (1,53 ml, 11 mmol), DMAP (122 mg, 1 mmol) und BHT (10 mg) in CH₂Cl₂ (16 ml) wurde unter Rühren eine Lösung von Acrylsäurechlorid (996 mg, 11 mmol) in CH₂Cl₂ (4 ml) über einen Zeitraum von 1 h so zugetropft, dass die Temperatur der Reaktionsmischung zwischen -5 and 0 °C blieb. Nach Erwärmen auf Raumtemperatur wurde 16 h gerührt und dann das Lösungsmittel im Vakuum abdestilliert. Der erhaltene Rückstand wurde mit einer Mischung aus MTBE (50 ml) und Wasser (25 ml) versetzt. Diese Mischung wurde mit 10%iger H₂SO₄ auf einen pH-Wert von ~5 eingestellt. Nach Abtrennen der organischen Phase wurde die wässrige Phase mit MTBE (3 × 20 ml) extrahiert. Die vereinigten organischen Phasen wurden mit Wasser (20 ml) und gesättigter NaCl-Lösung (2 × 25 ml) gewaschen und über wasserfreiem Magnesiumsulfat getrocknet. Nach Abdestillieren des Lösungsmittels erhielt man ein Öl, das durch Flash-Chromatographie (Kieselgel (100 ml), Elutionsmittel Chloroform/MTBE 6:1 bis 3:1) gereinigt wurde, wobei 3,19 g (71%) [10-(N-Acryloyl-N-methoxyamino)decyl]phosphorsäuredi-tert-butyl ester **9** als schwach gelbliches Ö1 erhalten wurden. ¹H-NMR (CDCl₃, 250 MHz): δ = 1,22-1,39 (m, 12 H), 1,47 (s, 18 H), 1,58-1,69 (m, 4 H), 3,65 (t, J = 7,4 Hz, 2 H), 3,69 (s, 3 H), 3,93 (q, J = 6,6 Hz, 2 H), 5,74 (dd, *J* = 10,3, 2,0 Hz, 1 H), 6,42 (dd, J = 17,1, 2,0 Hz, 1 H), 6,72 (dd, J = 17,1, 10,3 Hz, 1 H) ppm. ¹³C-NMR (CDCl₃, 75,5 MHz): δ = 25,6 (CH₂), 26,7 (CH₂), 27,0 (CH₂), 29, 1 (CH₂), 29,2 (CH₂), 29,3 (CH₂), 29,4 (CH₂), 29,8 (CH₃), 29,9 (CH₃), 30,2 (CH₂), 30,3 (CH₂), 62,1 (CH₃), 66,8 (CH₂), 66,9 (CH₂), 81,8 (C), 81,9 (C), 126,2 (CH), 128,9 (CH₂), 166,0 (C) ppm.

### 5. Stufe: [10-(N-Acryloyl-N-methoxyamino)decyl]phosphorsäure (10) :

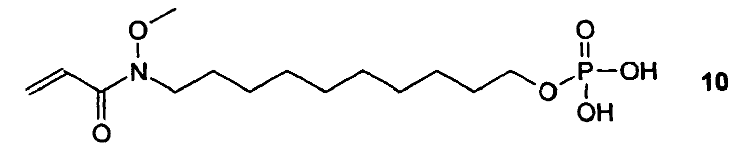

[10-(*N*-Acryloyl-*N*-methoxyamino)decyl]phosphorsäuredi-*tert-*butyl ester **9** (4,5 g, 10 mmol) wurde in Tetrachlorkohlenstoff (20 ml) gelöst und dazu wurde Trifluoressigsäure (3,07 g, 26,9 mmol) zugegeben. Das Reaktionsgemisch wurde am Rotationsverdampfer (600-630 mbar) 1 h auf 50 °C erwärmt und anschliessend wurde der Druck im Verlauf von 1 h auf 5 mbar verringert. Der Rückstand wurde dann im Feinvakuum (ca. 0,03 mbar) bis zur Gewichtskonstanz getrocknet, wobei 2,84 g (84%) [10-(N-Acryloyl-N-methoxyamino)decyl]phosphorsäure **10** als gelbliches Ö1 erhalten wurden. ¹H-NMR (CDCl₃, 300 MHz): δ = 1,28-1,37 (m, 12 H), 1,64-1,67 (m, 4 H), 3,67-3,71 (m, 5 H), 4,01 (q, 2 H), 5,83 (dd, 1 H), 6,42 (dd, 1 H), 6,46 (m, 1 H), 11,56 (s, 2 H) ppm. ³¹P-NMR (DMSO-d₆, 162 MHz,): δ = 2,58 ppm.

### Beispiel 3: Radikalische Polymerisation des Bis(meth)acrylamides 4

In Schlenkgefässen wurden Lösungen aus 30% 1,6-Bis(*N*-acryloyl-N-methoxyamino)hexan **4** aus Beispiel 1 bzw. aus 30% Glycerindimethacrylat als Vergleichsbeispiel und 1 Masse-% 2,2'-Azobis-(isobutyronitril) (Initiator) in Chlorbenzol hergestellt und mittels Durchleiten von Argon entgast. Danach wurden die Polymerisationsansätze in einem Thermostat auf 65 °C erwärmt. Die Zeit, in der sich ein dreidimensionales standfestes Gel ausbildet, wurde als Gelzeit bestimmt.

| **Vernetzer** | **Gelzeit** |
|---|---|
| 1,6-Bis(N-acryloyl-*N*-methoxyamino) hexan **4** | 80 s |
| Glycerindimethacrylat (Vergleichsbeispiel) | 7 min |

Das Beispiel belegt die hohe Reaktivität der Weinrebvernetzer in der radikalischen Polymerisation im Vergleich zu kommerziellen Dimethacrylaten.

### Beispiel 4: [10-(N-Acryloyl-N-methoxyamino)decyl]phosphorsäure 10 aus Beispiel 3 enthaltendes Dentinadhäsiv

Zur Untersuchung der Dentinhaftung auf Rinderzahndentin wurde ein Adhäsiv folgender Zusammensetzung (Angabe in Gew.-%) hergestellt:

| | |
|---|---|
| Stark saures Adhäsivmonomer **10**^{a)}**:** | 11,1 % |
| Glycerindimethacrylat: | 11,0 % |
| 2-Hydroxyethylmethacrylat: | 20,0 % |
| Ethanol: | 24,0 % |
| Bis-GMA: | 33,1 % |
| Photoinitiator: | 0,8 % |

### a) [10-(N-Acryloyl-N-methoxyamino)decyl]phosphorsäure

Rinderzähne wurden so in einen Kunststoffzylinder eingebettet, dass sich das Dentin und der Kunststoff in einer Ebene befanden. Nach 15 s Ätzung mit 37%iger Phosphorsäure wurde gründlich mit Wasser abgespült. Durch die Säureätzung werden die Dentintubuli geöffnet. Dann wurde mit einem Microbrush eine Schicht Adhäsiv obiger Zusammensetzung aufgepinselt, diese mit dem Luftbläser zur Entfernung des Lösungsmittels kurz verblasen und 40 s mit einer Halogenlampe (Astralis 7, Ivoclar Vivadent AG) belichtet. Auf die Adhäsivschicht wurde ein Kompositzylinder aus Tetric^{®} Ceram (Ivoclar Vivadent AG) in zwei Schichten von je 1-2 mm durch jeweils 40 s Belichtung mit der Halogenlampe Astralis 7 polymerisiert. Anschliessend wurden die Prüfkörper 24 h bei 37 °C in Wasser gelagert und die Scherhaftfestigkeit zu 17,0 MPa bestimmt, was für eine nicht optimierte Adhäsivzusammensetzung einen sehr hohen Dentinhaftwert darstellt.

### Beispiel 5: [10-(N-Acryloyl-N-methoxyamino)decyl] phosphorsäure 10 aus Beispiel 3 enthaltendes selbsthaftendes Komposit

Zur Untersuchung der Dentinhaftung auf Rinderzahndentin wurde ein Komposit folgender Zusammensetzung (Angabe in Gew.-%) hergestellt:

| | |
|---|---|
| Stark saures Adhäsivmonomer **10**^{a)}**:** | 3,0 % |
| UDMA | 5,7 % |
| 2-Acetoacetoxyethylmethacrylat: | 3,0 % |
| Bis-GMA: | 8,0 % |
| Photoinitiator^{b)}: | 0,3 % |
| Füllstoffe^{c)}: | 80,0 % |

| | |
|---|---|
| ^{a)} [10-(*N*-Acryloyl-*N*-methoxyamino)decyl]phosphorsäure ^{b)} Mischung aus 0,1 % Campherchinon und 0,2 % 4-Dimethylaminobenzoesäureethylester ^{c)} Mischung aus 39 Gew.-% silanisiertem Ba-Al-Silikat-Glasfüller und 41 Gew.-% Isofüller. | |

Analog zum Beispiel 4 wurden eingebettete Rinderzähne verwendet. Die Dentinoberfläche wurde mit Schleifpapier erst fein (P120) dann sehr fein (P600) angeschliffen. Auf diese Dentinfläche wurde direkt eine dünne Schicht des voranstehend beschriebenen Komposites aufgetragen und darauf ein Kompositzylinder aus Tetric^{®} Ceram (Ivoclar Vivadent AG) mit einem Durchmesser von 4 mm aufgedrückt. Anschliessend wurde von allen Seiten 4 x 20 s mit der Halogenlampe Astralis 7 belichtet. Dann wurden die Prüfkörper 24 h bei 37 °C in Wasser gelagert und die Scherhaftfestigkeit zu 7,2 MPa bestimmt, was für die Selbsthaftung eines Komposites einen ausgezeichneten Wert darstellt.

## Patentansprüche

1. Dentalmaterial, **dadurch gekennzeichnet, dass** es mindestens ein polymerisationsfähiges N,O-funktionalisiertes Acrylsäure-hydroxamid der allgemeinen Formel (I) 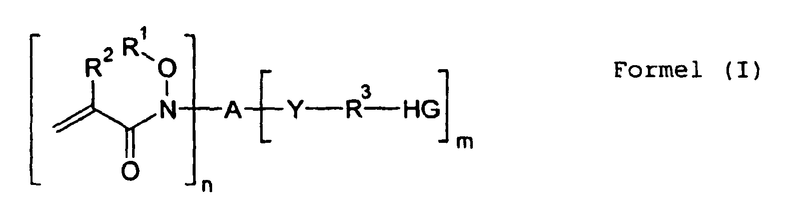 enthält, in der
A ein n+m-wertiger linearer oder verzweigter aliphatischer C₁- bis C₅₀-Rest, bei dem die Kohlenstoffkette durch O, S, -CO-O-, CO-NH, O-CO-NH oder NH-CO-NH unterbrochen sein kann, ein n+m-wertiger aromatischer C₆- bis C₁₈-Rest oder einem n+m-wertiger cycloaliphatischer bzw. heterocyclischer C₃- bis C₁₈-Rest ist, wobei die Reste einen oder mehrere Substituenten tragen können,
Y entfällt, O, S, eine Ester-, Amid- oder Urethangruppe ist,
R¹ Wasserstoff, ein aliphatischer C₁- bis C₂₀-Alkyl- oder C₃- bis C₈-Cycloalkylrest ist, der einen oder mehrere Substitu- enten tragen kann,
R² H oder ein C₁- bis C₁₀-Alkylrest ist,
R³ entfällt oder ein C₁- bis C₁₆-Alkylenrest ist, der durch O unterbrochen sein kann,
HG entfällt, -COOH, -P=O(OH)₂; -P=O(OH)(OR⁴); -O-P=O(OH)₂, -SO₂OH oder -O-P=O(OH)(OR⁴) ist,
R⁴ ein C₁- bis C₁₅-Alkylrest, Phenyl- oder Benzyl-Rest ist und
n eine Zahl von 1 bis 5 und
m eine Zahl von 0 bis 3 ist.

2. Dentalmaterial nach Anspruch 1, **dadurch gekennzeichnet, dass** der eine oder die mehreren Substituenten an dem Rest A ausgewählt sind aus der Gruppe bestehend aus C₁- bis C₅-Alkylgruppen, Cl, Br und OH.

3. Dentalmaterial nach Anspruch 1 oder Anspruch 2, **dadurch gekennzeichnet, dass** der eine oder die mehreren Substituenten an dem Rest R¹ ausgewählt sind aus der Gruppe bestehend aus Cl, Br und OH.

4. Dentalmaterial nach einem der vorhergehenden Ansprüche, **dadurch gekennzeichnet, dass**
A ein n+m-wertiger linearer oder verzweigter aliphati- scher C₁- bis C₃₀-Rest, bei dem die Kohlenstoffkette durch O, -CO-O- oder O-CO-NH unterbrochen sein kann, ein n+m-wertiger aromatischer C₆- bis C₁₀-Rest oder ein n+m-wertiger cycloaliphatischer C₃- bis C₁₈-Rest ist, wobei die Reste einen oder mehrere Substituenten tragen können,
Y entfällt, O, eine Ester- oder Urethangruppe ist,
R¹ ein aliphatischer C₁- bis C₈-Alkyl- oder Cycloalkylrest ist,
R² H oder CH₃ ist,
R³ entfällt oder ein C₁- bis C₁₂-Alkylenrest, der durch O unterbrochen sein kann, ist,
HG entfällt, -COOH, -P=O(OH)₂, -O-P=O(OH)₂, -SO₂OH oder - O-P=O(OH)(OR⁴) ist,
R⁴ ein C₁- bis C₄-Alkylrest ist,
n eine Zahl von 1 bis 3 ist und
m eine Zahl von 0 bis 2 ist.

5. Dentalmaterial nach Anspruch 4, **dadurch gekennzeichnet, dass** der eine oder die mehreren Substituenten an dem Rest A ausgewählt sind aus der Gruppe bestehend aus C₁- bis C₃-Alkylgruppen und OH.

6. Dentalmaterial nach einem der vorhergehenden Ansprüche, **dadurch gekennzeichnet, dass**
A ein n+m-wertiger linearer oder verzweigter aliphati- scher C₁- bis C₁₂-Rest, ein n+m-wertiger cycloaliphati- scher C₆- bis C₁₂-Rest oder ein n+m-wertiger aromati- scher C₆- bis C₁₀-Rest ist,
Y entfällt oder eine Ester-Gruppe ist,
R¹ C₁- bis C₄-Alkyl ist,
R² H oder CH₃ ist,
R³ entfällt oder C₄- bis C₈-Alkylen-Rest ist, der durch O unterbrochen sein kann,
HG entfällt, -COOH, -P=O(OH)₂, -OP=O(OH)₂, -OP=O(OH)(OC₂H₅) oder -SO₂OH ist
n 1, 2 oder 3 und
m 0 oder 1 ist.

7. Dentalmaterial nach Anspruch 6, **dadurch gekennzeichnet, dass**
A ein n+m-wertiger linearer oder verzweigter aliphati- scher C₁- bis C₅-Rest, ein n+m-wertiger cycloaliphati- scher C₆-Rest oder ein n+m-wertiger aromatischer C₆-Rest ist,
Y entfällt oder -COO- ist,
R¹ Methyl, Ethyl oder Propyl ist,
R² H oder CH₃ ist,
R³ entfällt oder Pentylen ist,
HG entfällt, -COOH, -P=O(OH)₂, -OP=O(OH)₂, -OP=O (OH) (OC₂H₅) oder -SO₂OH ist,
n 1, 2 oder 3 und
m 0 oder 1 ist.

8. Dentalmaterial nach einem der vorhergehenden Ansprüche, **dadurch gekennzeichnet, dass** es mindestens einen Polymerisationsinitiator enthält.

9. Dentalmaterial nach einem der vorhergehenden Ansprüche, **dadurch gekennzeichnet, dass** es mindestens ein weiteres polymerisierbares Verdünnermonomer enthält.

10. Dentalmaterial nach einem der vorhergehenden Ansprüche, **dadurch gekennzeichnet, dass** es mindestens ein weiteres Vernetzermonomer enthält.

11. Dentalmaterial nach einem der vorhergehenden Ansprüche, **dadurch gekennzeichnet, dass** es mindestens einen Füllstoff enthält.

12. Dentalmaterial nach einem der vorhergehenden Ansprüche, **dadurch gekennzeichnet, dass** es mindestens ein Lösungsmittel enthält.

13. Dentalmaterial nach einem der vorhergehenden Ansprüche, **dadurch gekennzeichnet, dass** es bezogen auf die Gesamtmasse des Materials
(a) 1 bis 95 Gew.-% Acrylamid der allgemeinen Formel (I),
(b) 0 bis 70 Gew.-% Verdünnermonomer,
(c) 0 bis 70 Gew.-% Vernetzermonomer,
(d) 0,1 bis 5,0 Gew.-% Polymerisationsinitiator und
(e) 0 bis 80 Gew.-% Füllstoff,
(f) 0 bis 70 Gew. -% Lösungsmittel,
(g) 0 bis 70 Gew.-% Adhäsivmonomer enthält.

14. Zusammensetzung nach Anspruch 13, **dadurch gekennzeichnet, dass**
(a) 5 bis 70 Gew.-% Acrylamid der allgemeinen Formel (I),
(b) 0 bis 40 Gew.-% Verdünnermonomer,
(c) 0 bis 40 Gew.-% Vernetzermonomer,
(d) 0,2 bis 2,0 Gew. -% Polymerisationsinitiator und
(e) 0 bis 50 Gew.-% Füllstoff,
(f) 0 bis 50 Gew.-% Lösungsmittel,
(g) 0 bis 50 Gew.-% Adhäsivmonomer enthält.

15. Amid der allgemeinen Formel (I) wie in einem der vorhergehenden Ansprüche 1 bis 7 definiert zur Verwendung als Dentalmaterial.

16. Verwendung eines Amids der allgemeinen Formel (I) wie in einem der vorhergehenden Ansprüche 1 bis 7 definiert zur Herstellung eines Dentalmaterials.

17. Amid oder Verwendung nach einem der vorhergehenden Ansprüche 15 oder 16, **dadurch gekennzeichnet, dass** das Dentalmaterial eine Zusammensetzung wie in einem der Ansprüche 8 bis 14 definiert aufweist.

18. Amid oder Verwendung nach einem der vorhergehenden Ansprüche 15 bis 17, **dadurch gekennzeichnet, dass** das Dentalmaterial ein dentales Adhäsiv, Beschichtungsmaterial, Füllungsmaterial oder dentaler Zement ist.

## Claims

1. Dental material, **characterized in that** it contains at least one polymerizable N,O-functionalized acrylic acid hydroxamide of the general Formula (I) 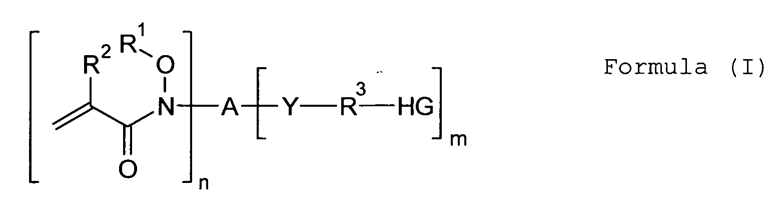 in which
A is an n+m-valent linear or branched aliphatic C₁ to C₅₀ radical, in which the carbon chain can be interrupted by O, S, -CO-O-, CO-NH, O-CO-NH or NH-CO-NH, an n+m-valent aromatic C₆ to C₁₈ radical or an n+m-valent cycloaliphatic or heterocyclic C₃ to C₁₈ radical, wherein the radicals can carry one or more substituents,
Y is not present, or is O, S, an ester, amide or urethane group,
R¹ is hydrogen, an aliphatic C₁ to C₂₀ alkyl or C₃ to C₈ cycloalkyl radical which can carry one or more substituents,
R² is H or a C₁ to C₁₀ alkyl radical,
R³ is not present or is a C₁ to C₁₆ alkylene radical which can be interrupted by O,
HG is not present, or is -COOH, -P=O(OH)₂; -P=O(OH)(OR⁴) ; -O-P=O(OH)₂, -SO₂OH or -O-P=O(OH)(OR⁴),
R⁴ is a C₁ to C₁₅ alkyl radical, phenyl or benzyl radical and
n is a number from 1 to 5 and
m a number from 0 to 3.

2. Dental material according to claim 1, **characterized in that** the one or more substituents at the radical A are selected from the group consisting of C₁ to C₅ alkyl groups, Cl, Br and OH.

3. Dental material according to claim 1 or claim 2, **characterized in that** the one or more substituents at the radical R¹ are selected from the group consisting of Cl, Br and OH.

4. Dental material according to one of the previous claims, **characterized in that**
A is an n+m-valent linear or branched aliphatic C₁ to C₃₀ radical, in which the carbon chain can be interrupted by O, -CO-O- or O-CO-NH, an n+m-valent aromatic C₆ to C₁₀ radical, or an n+m-valent cycloaliphatic C₃ to C₁₈ radical, wherein the radicals can carry one or more substituents,
Y is not present, is O, an ester or urethane group,
R¹ is an aliphatic C₁ to C₈ alkyl or cycloalkyl radical,
R² is H or CH₃,
R³ is not present or is a C₁ to C₁₂ alkylene radical which can be interrupted by O,
HG is not present, or is -COOH, -P=O(OH)₂, -O-P=O(OH)₂, -SO₂OH or -O-P=O(OH)(OR⁴),
R⁴ is a C₁ to C₄ alkyl radical,
n is a number from 1 to 3 and
m is a number from 0 to 2.

5. Dental material according to claim 4, **characterized in that** the one or more substituents at the radical A are selected from the group consisting of C₁ to C₃ alkyl groups and OH.

6. Dental material according to one of the previous claims, **characterized in that**
A is an n+m-valent linear or branched aliphatic C₁ to C₁₂ radical, an n+m-valent cycloaliphatic C₆ to C₁₂ radical or an n+m-valent aromatic C₆ to C₁₀ radical,
Y is not present or is an ester group,
R¹ is a C₁ to C₄ alkyl,
R² is H or CH₃,
R³ is not present or is a C₄ to C₈ alkylene radical which can be interrupted by O,
HG is not present, or is -COOH, -P=O(OH)₂, -OP=O(OH)₂, -OP=O(OH)(OC₂H₅) or -SO₂OH,
n is 1, 2 or 3 and
m is 0 or 1.

7. Dental material according to claim 6, **characterized in that**
A is an n+m-valent linear or branched aliphatic C₁ to C₅ radical, an n+m-valent cycloaliphatic C₆ radical or an n+m-valent aromatic C₆ radical,
Y is not present or is -COO-,
R¹ is methyl, ethyl or propyl,
R² is H or CH₃,
R³ is not present or is pentylene,
HG is not present, or is -COOH, -P=O(OH)₂, -OP=O(OH)₂, -OP=O (OH) (OC₂H₅) or -SO₂OH,
n is 1, 2 or 3 and
m is 0 or 1.

8. Dental material according to one of the previous claims, **characterized in that** it contains at least one polymerization initiator.

9. Dental material according to one of the previous claims, **characterized in that** it contains at least one further polymerizable diluent monomer.

10. Dental material according to one of the previous claims, **characterized in that** it contains at least one further cross-linker monomer.

11. Dental material according to one of the previous claims, **characterized in that** it contains at least one filler.

12. Dental material according to one of the previous claims, **characterized in that** it contains at least one solvent.

13. Dental material according to one of the previous claims, **characterized in that**, relative to the total mass of the material, it contains
(a) 1 to 95 wt.-% acrylamide of the general Formula (I),
(b) 0 to 70 wt.-% diluent monomer,
(c) 0 to 70 wt.-%, cross-linker monomer,
(d) 0.1 to 5.0 wt.-% polymerization initiator and
(e) 0 to 80 wt.-% filler,
(f) 0 to 70 wt.-% solvent,
(g) 0 to 70 wt.-% adhesive monomer.

14. Composition according to claim 13, **characterized in that** it contains
(a) 5 to 70 wt.-% acrylamide of the general Formula (I),
(b) 0 to 40 wt.-% diluent monomer,
(c) 0 to 40 wt.-% cross-linker monomer,
(d) 0.2 to 2.0 wt.-% polymerization initiator and
(e) 0 to 50 wt.-% filler,
(f) 0 to 50 wt.-% solvent,
(g) 0 to 50 wt.-% adhesive monomer.

15. Amide of the general Formula (I) as defined in one of the previous claims 1 to 7 for use as a dental material.

16. Use of an amide of the general Formula (I) as defined in one of the previous claims 1 to 7 for the preparation of a dental material.

17. The amide or use according to one of the previous claims 15 or 16, **characterized in that** the dental material has a composition as defined in one of the claims 8 to 14.

18. The amide or use according to one of the previous claims 15 to 17, **characterized in that** the dental material is a dental adhesive, coating material, filling material or dental cement.

## Revendications

1. Matériau dentaire, **caractérisé en ce qu'**il contient au moins un hydroxyamide d'acide acrylique N,O-fonctionnalisé et polymérisable, de formule générale (I) : 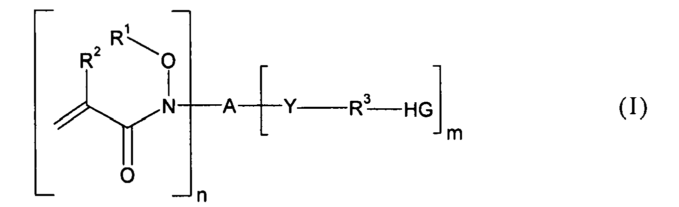 dans laquelle
- A représente un reste aliphatique en C₁₋₅₀, à chaîne linéaire ou ramifiée et de valence n+m, dont la chaîne d'atomes de carbone peut être interrompue par un ou des chaînon(s) symbolisé(s) par -O-, -S-, -CO-O-, -CO-NH-, -O-CO-NH- ou -NH-CO-NH-, un reste aromatique en C₆₋₁₈ de valence n+m, ou un reste cycloaliphatique ou hétérocyclique en C₃₋₁₈ de valence n+m, lesquels restes peuvent porter un ou plusieurs substituant(s) ;
- Y ne représente rien ou représente un atome d'oxygène ou de soufre ou un groupe ester, amide ou uréthane ;
- R¹ représente un atome d'hydrogène ou un groupe aliphatique alkyle en C₁₋₂₀ ou cycloalkyle en C₃₋₈, qui peut porter un ou plusieurs substituant(s) ;
- R² représente un atome d'hydrogène ou un groupe alkyle en C₁₋₁₀ ;
- R³ ne représente rien ou représente un groupe alcanediyle en C₁₋₁₆, dont la chaîne peut être interrompue par un ou des atome(s) d'oxygène ;
- HG ne représente rien ou représente un groupe symbolisé par -COOH, -P(=O)(OH)₂, -P(=O)(OH)(OR⁴), -O-P(=O)(OH)₂, -SO₂(OH) ou -O-P(=O)(OH)(OR⁴),
où R⁴ représente un groupe alkyle en C₁₋₁₅, phényle ou benzyle ;
- l'indice n est un nombre qui vaut de 1 à 5 ;
- et l'indice m est un nombre qui vaut de 0 à 3.

2. Matériau dentaire conforme à la revendication 1, **caractérisé en ce que** le ou les substituant(s) portés par le reste A est ou sont choisi(s) dans l'ensemble formé par les groupes alkyle en C₁₋₅, les atomes de chlore et de brome et le groupe hydroxyle.

3. Matériau dentaire conforme à la revendication 1 ou 2, **caractérisé en ce que** le ou les substituant(s) portés par le groupe R¹ est ou sont choisi(s) dans l'ensemble formé par les atomes de chlore et de brome et le groupe hydroxyle.

4. Matériau dentaire conforme à l'une des revendications précédentes, **caractérisé en ce que** :
- A représente un reste aliphatique en C₁₋₃₀, à chaîne linéaire ou ramifiée et de valence n+m, dont la chaîne d'atomes de carbone peut être interrompue par un ou des chaînon(s) symbolisé(s) par -O-, -CO-O- ou -O-CO-NH-, un reste aromatique en C₆₋₁₀ de valence n+m, ou un reste cycloaliphatique en C₃₋₁₈ de valence n+m, lesquels restes peuvent porter un ou plusieurs substituant(s) ;
- Y ne représente rien ou représente un atome d'oxygène ou un groupe ester ou uréthane ;
- R¹ représente un groupe aliphatique alkyle en C₁₋₈ ou cycloalkyle ;
- R² représente un atome d'hydrogène ou un groupe méthyle ;
- R³ ne représente rien ou représente un groupe alcanediyle en C₁₋₁₂, dont la chaîne peut être interrompue par un ou des atome(s) d'oxygène ;
- HG ne représente rien ou représente un groupe symbolisé par -COOH, -P(=O)(OH)₂, -O-P(=O)(OH)₂, -SO₂(OH) ou -O-P(=O)(OH)(OR⁴),
où R⁴ représente un groupe alkyle en C₁₋₄ ;
- l'indice n est un nombre qui vaut de 1 à 3 ;
- et l'indice m est un nombre qui vaut de 0 à 2.

5. Matériau dentaire conforme à la revendication 4, **caractérisé en ce que** le ou les substituant(s) portés par le reste A est ou sont choisi(s) parmi les groupes alkyle en C₁₋₃ et le groupe hydroxyle.

6. Matériau dentaire conforme à l'une des revendications précédentes, **caractérisé en ce que** :
- A représente un reste aliphatique en C₁₋₁₂, à chaîne linéaire ou ramifiée et de valence n+m, un reste cycloaliphatique en C₆₋₁₂ de valence n+m, ou un reste aromatique en C₆₋₁₀ de valence n+m ;
- Y ne représente rien ou représente un groupe ester ;
- R¹ représente un groupe alkyle en C₁₋₄ ;
- R² représente un atome d'hydrogène ou un groupe méthyle ;
- R³ ne représente rien ou représente un groupe alcanediyle en C₄₋₈, dont la chaîne peut être interrompue par un ou des atome(s) d'oxygène ;
- HG ne représente rien ou représente un groupe symbolisé par -COOH, -P(=O)(OH)₂, -O-P(=O)(OH)₂, -O-P(=O)(OH)(OC₂H₅) ou -SO₂(OH) ;
- l'indice n vaut 1, 2 ou 3 ;
- et l'indice m vaut 0 ou 1.

7. Matériau dentaire conforme à la revendication 6, **caractérisé en ce que**
- A représente un reste aliphatique en C₁₋₅, à chaîne linéaire ou ramifiée et de valence n+m, un reste cycloaliphatique en C₆ de valence n+m, ou un reste aromatique en C₆ de valence n+m ;
- Y ne représente rien ou représente un groupe symbolisé par -COO- ;
- R¹ représente un groupe méthyle, éthyle ou propyle ;
- R² représente un atome d'hydrogène ou un groupe méthyle ;
- R³ ne représente rien ou représente un groupe pentanediyle ;
- HG ne représente rien ou représente un groupe symbolisé par -COOH, -P(=O)(OH)₂, -O-P(=O)(OH)₂, -O-P(=O)(OH)(OC₂H₅) ou -SO₂(OH) ;
- l'indice n vaut 1, 2 ou 3 ;
- et l'indice m vaut 0 ou 1.

8. Matériau dentaire conforme à l'une des revendications précédentes, **caractérisé en ce qu'**il contient au moins un amorceur de polymérisation.

9. Matériau dentaire conforme à l'une des revendications précédentes, **caractérisé en ce qu'**il contient au moins un autre monomère polymérisable servant de diluant.

10. Matériau dentaire conforme à l'une des revendications précédentes, **caractérisé en ce qu'**il contient au moins un autre monomère servant d'agent de réticulation.

11. Matériau dentaire conforme à l'une des revendications précédentes, **caractérisé en ce qu'**il contient au moins une charge.

12. Matériau dentaire conforme à l'une des revendications précédentes, **caractérisé en ce qu'**il contient au moins un solvant.

13. Matériau dentaire conforme à l'une des revendications précédentes, **caractérisé en ce qu'**il contient, par rapport au poids total du matériau :
a) 1 à 95 % en poids d'un dérivé d'acrylamide de formule générale (I),
b) 0 à 70 % en poids d'un monomère servant de diluant,
c) 0 à 70 % en poids d'un monomère servant d'agent de réticulation,
d) 0,1 à 5,0 % en poids d'un amorceur de polymérisation,
e) 0 à 80 % en poids d'une charge,
f) 0 à 70 % en poids d'un solvant,
g) et 0 à 70 % en poids d'un monomère servant d'agent d'adhésion.

14. Composition conforme à la revendication 13, **caractérisée en ce qu'**elle contient :
a) 5 à 70 % en poids d'un dérivé d'acrylamide de formule générale (I),
b) 0 à 40 % en poids d'un monomère servant de diluant,
c) 0 à 40 % en poids d'un monomère servant d'agent de réticulation,
d) 0,2 à 2,0 % en poids d'un amorceur de polymérisation,
e) 0 à 50 % en poids d'une charge,
f) 0 à 50 % en poids d'un solvant,
g) et 0 à 50 % en poids d'un monomère servant d'agent d'adhésion.

15. Amide de formule générale (I), du type défini dans l'une des revendications 1 à 7 précédentes, pour son utilisation en tant que matériau dentaire.

16. Utilisation d'un amide de formule générale (I), du type défini dans l'une des revendications 1 à 7 précédentes, en vue de la fabrication d'un matériau dentaire.

17. Amide ou utilisation, conforme à la revendication 15 ou 16 précédente, **caractérisé**(e) en ce que le matériau dentaire présente une composition du type défini dans l'une des revendications 8 à 14.

18. Amide ou utilisation, conforme à l'une des revendications 15 à 17 précédentes, **caractérisé**(e) en ce que le matériau dentaire est un adhésif dentaire, un matériau de revêtement, un matériau de remplissage ou un ciment dentaire.
